Europäisches Patentamt

European Patent Office

Office européen des brevets

(11) Veröffentlichungsnummer: **0 491 279 A1**

# EUROPÄISCHE PATENTANMELDUNG

(21) Anmeldenummer: **91121322.1**

(51) Int. Cl.⁵: **A61L 2/16**

(22) Anmeldetag: **12.12.91**

(30) Priorität: **17.12.90 DE 4040316**

(43) Veröffentlichungstag der Anmeldung:
**24.06.92 Patentblatt 92/26**

(84) Benannte Vertragsstaaten:
**AT BE CH DE DK ES FR GB GR IT LI LU NL SE**

(71) Anmelder: **Hewing GmbH
Waldstrasse 3
W-4434 Ochtrup(DE)**

(72) Erfinder: **Olbrich, Kurt
Hardtstrasse 11
W-6121 Mossautal-Hiltersklingen(DE)**
Erfinder: **Höffker, Bruno
Johannesweg 8
W-4440 Rheine-Mesum(DE)**

(74) Vertreter: **Busse & Busse Patentanwälte
Postfach 1226 Grosshandelsring 6
W-4500 Osnabrück(DE)**

(54) Verfahren zur keimwachstumshemmenden Behandlung von Oberflächen von technischen Gegenständen und Hohlkörper zur Begrenzung flüssiger oder gasförmiger Medien.

(57) Die Erfindung bezieht sich auf ein Verfahren zur keimwachstumshemmenden Behandlung von Oberflächen von technischen Gegenständen, wobei insbesondere Hohlkörper wie Behälter, Leitungen, Ventile oder dgl. sanitärtechnische Bauteile umfaßt sind, die Oberflächen zur Begrenzung strömender oder ruhender, Keime enthaltender Medien aufweisen. Um insbesondere ein Verfahren zu schaffen, das mit geringem Aufwand das Keimwachstum in mit den behandelten Oberflächen in Berührung gelangenden flüssigen und/oder gasförmigen Medien einschränkt, werden bei Gegenständen mit Oberflächen zur Begrenzung strömender oder ruhender flüssiger oder gasförmiger, Keime enthaltender Medien diese Oberflächen halogeniert.

Die Erfindung bezieht sich auf ein Verfahren zur keimwachstumshemmenden Behandlung von Oberflächen von technischen Gegenständen gemäß dem Oberbegriff des Anspruchs 1 sowie auf einen Hohlkörper gemäß dem Oberbegriff des Anspruchs 7.

Um das Wachstum von Bakterien, Sporen und Pilzen einzudämmen bzw. vollständig zu verhindern, ist es bekannt, von Befall bedrohte Bereiche technischer Oberflächen mit bakteriziden bzw. fungiziden Anstrichstoffen zu beschichten. Derartige antimikrobielle Beschichtungen sind mit erheblichem Aufwand verbunden und nur für frei zugängliche Oberflächen geeignet, da sie von Zeit zu Zeit erneuert oder bei mechanischen Beschädigungen ausgebessert werden müssen. Mit Hilfe der in den Anstrichstoffen befindlichen bakterizid oder fungizid wirkenden Bestandteile wird das Keimwachstum an der entsprechenden Oberfläche gehemmt, um den darunter liegenden Körper zu schützen.

Der Erfindung liegt die Aufgabe zugrunde, ein Verfahren zur keimwachstumshemmenden Behandlung von Oberflächen von technischen Gegenständen zu schaffen, das zur Anwendung an schwer oder im Betrieb nicht mehr zugänglichen Oberflächen, insbesondere solchen von Hohlkörpern, geeignet ist und mit wenig Aufwand das Keimwachstum in mit den behandelten Oberflächen in Berührung gelangenden flüssigen und/oder gasförmigen Medien einschränkt. Ferner liegt ihr die Aufgabe zugrunde, einen Hohlkörper derart auszugestalten, daß seine mit strömenden oder ruhenden flüssigen und/oder gasförmigen Medien in Berührung gelangenden Oberflächen eine wachstumshemmende Wirkung auf in den Medien enthaltene Keime ausüben.

Die Erfindung löst diese Aufgabe durch ein Verfahren gemäß Anspruch 1 sowie einen Hohlkörper gemäß Anspruch 8. Hinsichtlich wesentlicher weiterer Ausgestaltungen wird auf die Ansprüche 2 bis 7 bzw. 9 bis 14 verwiesen.

Die Erfindung schafft auf besonders einfache und effektive Weise die Möglichkeit zur Hemmung von Keimwachstum in fließenden oder ruhenden flüssigen oder gasförmigen Medien, die mit technischen Oberflächen in Berührung stehen, wie das insbesondere bei Hohlkörpern, z.B. Behältern oder Rohrleitungen bzw. Kanälen sowie Armaturen, der Fall ist. Überraschenderweise wird durch eine Halogenierung der Oberfläche derartiger Bauteile das Wachstum insbesondere von Legionellen gehemmt, was für Heizungs- und/oder Lüftungsanlagen von erheblicher Bedeutung ist, deren Kanäle bzw. Leitungen in steigendem Umfange aus verschiedensten Werkstoffen bestehende Begrenzungsflächen für die Wärmeträger- bzw. Belüftungsmedien darbieten. Insbesondere eine Fluorierung,deren Durchführung mit einfachen Mitteln möglich ist, erbringt eine signifikante Wachstumshemmung, wie sie in Fällen, in denen das Medium, z.B. durch seine Temperatur, einem starken Keimwachstum Vorschub leistet, besonders erwünscht ist.

Ganz besonders sind fluorierte Kunststoffoberflächen, vor allem solche aus vernetztem Polyäthylen, für Trink- und Brauchwasserversorgungen, Heizungs- und Kühlanlagen etc., geeignet, Gefahren aus Keimwachstum für Benutzer erheblich zu reduzieren.

Gerade in Warmwasser-Versorgungssystemen mit längeren Intervallen der Wasserentnahme, wie das in häufig weitverzweigten Netzen in Hotels, Heimen, Wohnkomplexen etc. der Fall sein kann, ist beispielsweise die Gefahr des Legionellen-Wachstums bei ruhendem Medium in Zweigleitungen bei entsprechenden Warmwassertemperaturen besonders hoch. Mit dem Einsatz von innenseitig fluorierten Rohren kann in solchen Leitungsnetzen das Legionellenwachstum auch in ruhendem Warmwasser gehemmt werden, wie Versuche ergeben haben, ohne daß damit eine sonstige Beeinträchtigung der Wasserqualität einhergeht. Dies gilt auch für Medien, die Klimaanlagen durchströmen, da die keimwachstumshemmende Wirkung auch in gasförmigen Medien feststellbar ist.

Das Halogenieren, insbesondere Fluorieren, von Kunststoffoberflächen von Gegenständen wie Leitungen und Behältern ist an sich bekannt (EP-OS-0 267 441), jedoch dient das bekannte Fluorieren zur Erhöhung der Diffusionsdichtigkeit (EP-A-0 267 441, DE-C-24 01 948, DE-A-35 29 870) bzw. des Randschichtwiderstandes (DE-C-26 44 508) oder der chemischen Beständigkeit (DE-A-38 21 015).

Bekannt ist auch die bakterizide bzw. fungizide Wirkung in chemischen Halogenverbindungen (DE-A-14 19 962, DE-A-27 56 031, DE-37 06 376), jedoch nur zu Konservierungs- bzw. Schutzzwecken, insbesondere von Holzkörpern, durch Zusatz in Außenbeschichtungen.

Bei der Durchführung des Verfahrens zur Halogenisierung der Begrenzungsflächen werden diese einem flüssigen oder gasförmigen Behandlungsmittel ausgesetzt, das beispielsweise 20 bis 30% Fluor enthält. Der Anteil an Halogen im Behandlungsmittel wird vom Material der Begrenzungsfläche des zu behandelnden Bauteils bestimmt, wobei für Oberflächen aus Kautschuk oder Gummi ähnlich niedrige Anteile wie bei Kunststoffen genügen, während höhere Anteile bei metallischen und keramischen Werkstoffen erforderlich sind. Die Dauer der Einwirkung des Halogens auf die Oberfläche kann über eine erhöhte Temperatur des Behandlungsmittels verkürzt werden, wobei Erhöhungen auf 90 bis 140°C in Abhängigkeit vom Material eine vorteilhafte Reduzierung der Behandlungszeit bewirken, insbesondere dann, wenn das Behandlungsverfahren mit einem Druck von 2,5 bis 6 bar auf das Behandlungsmittel realisiert wird.

Die Durchführung der erfindungsgemäßen Behandlung an keimwachstumsgefährdeten Bauteilbegrenzungsflächen ist nicht nur wirkungsvoll, wenn beispielsweise Rohre, Leitungen, keramisches Zubehör o.dgl. sanitäre Bauteile vor ihrem Einbau halogeniert werden, sondern auch dann, wenn Bauteile bereits in Gebrauch sind. Dann ist eine nachträgliche Behandlung der Begrenzungsflächen ebenfalls durchführbar, wozu die Begrenzungsfläche vorher einer gründlichen Säuberung, z.B. einem Entkalkungs und Entrostungsvorgang, zu unterziehen ist.

Bei einem Vorliegen unterschiedlicher Materialien an gleichzeitig zu behandelnden Begrenzungsflächen wird eine zuverlässige keimwachstumshemmende Behandlung erreicht, wenn die Behandlungsparameter auf das reaktionsträgste Material abgestimmt werden.

**Patentansprüche**

1. Verfahren zur keimwachstumshemmenden Behandlung von Oberflächen von technischen Gegenständen, **dadurch gekennzeichnet,** daß bei Gegenständen mit Oberflächen zur Begrenzung strömender oder ruhender flüssiger oder gasförmiger, Keime enthaltender Medien diese Oberflächen halogeniert werden.

2. Verfahren nach Anspruch 1, **dadurch gekennzeichnet,** daß die Oberflächen einer zeitlich begrenzten Einwirkung eines das Halogen enthaltenden flüssigen oder gasförmigen Behandlungsmittels ausgesetzt werden.

3. Verfahren nach Anspruch 1 oder 2, **dadurch gekennzeichnet,** daß die Oberflächen fluoriert werden.

4. Verfahren nach einem der Ansprüche 1 bis 3, **dadurch gekennzeichnet,** daß das Behandlungsmittel 20 bis 30% Fluor enthält.

5. Verfahren nach Anspruch 4, **dadurch gekennzeichnet,** daß das Behandlungsmittel eine Behandlungstemperatur von 90 bis 140° aufweist.

6. Verfahren nach Anspruch 4 oder 5, **dadurch gekennzeichnet,** daß das Behandlungsmittel unter einem Druck von 2,5 bis 6 bar gehalten wird.

7. Verfahren nach einem der Ansprüche 1 bis 6, **dadurch gekenzeichnet,** daß bei nachträglicher Halogenisierung von Oberflächen an in Gebrauch befindlichen Gegenständen die Oberflächen vor der Halogenisierung gesäubert, entkalkt und/oder entrostet werden.

8. Hohlkörper, insbesondere sanitärtechnische Bauteile wie Behälter, Leitungen, Ventile und Zubehörteile o.dgl., an denen Oberflächen zur Begrenzung strömender oder ruhender flüssiger oder gasförmiger, Keime enthaltender Medien ausgebildet sind, **dadurch gekennzeichnet,** daß die Bauteile zur keimwachstumshemmenden Wirkung an den Begrenzungsflächen halogeniert sind.

9. Hohlkörper nach Anspruch 8, **dadurch gekennzeichnet,** daß die Begrenzungsflächen der Bauteile fluoriert sind.

10. Hohlkörper nach Anspruch 8 oder 9, **dadurch gekennzeichnet,** daß zumindest die Begrenzungsflächen der Bauteile aus Stahl, Edelstahl, Messing, Kupfer o.dgl. halogenierten metallischen Werkstoffen bestehen.

11. Hohlkörper nach Anspruch 8 oder 9, **dadurch gekennzeichnet,** daß die Begrenzungsfläche aus halogeniertem nichtmetallischem Werkstoff besteht.

12. Hohlkörper nach Anspruch 11, **dadurch gekennzeichnet,** daß die Oberfläche aus halogeniertem Kunststoff, insbesondere vernetztem Polyäthylen, besteht.

13. Hohlkörper nach Anspruch 11, **dadurch gekennzeichnet,** daß die Oberfläche aus halogeniertem keramischen Material besteht.

14. Hohlkörper nach Anspruch 11, **dadurch gekennzeichnet,** daß die Oberfläche aus halogeniertem Kautschuk oder Gummi besteht.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | EP-A-0 253 037 (ATLANTIC PHARMACEUTICAL PRODUCTS LTD.) <br> * Seite 3, Zeile 46 - Zeile 56; Ansprüche * <br> * Seite 3, Zeile 1 - Zeile 2 * <br> --- | 1-12,14 | A61L2/16 |
| Y,D | EP-A-0 267 441 (HEWING GMBH & CO.) <br><br> * Seite 4, Spalte 5, Zeile 2 - Zeile 55; Ansprüche * <br> --- | 1-9, 11-12,14 | |
| Y | US-A-4 484 954 (G.TARANCON) <br> * Spalte 1, Zeile 55 - Zeile 60; Ansprüche * <br> * Spalte 2, Zeile 30 - Zeile 49 * <br> --- | 10 | |
| Y | US-A-3 992 221 (C.HOMSY) <br> * Spalte 1, Zeile 6 - Zeile 11 * <br> --- | 14 | |
| A | H.HUDEMANN UND W.WEUFFEN 'LEITFADEN DER DESINFECTIONSPRAXIS' <br> 1973 , JOHANN AMBROSIUS BARTH , LEIPZIG <br> * Seite 62 - Seite 64 * <br> --- | | |
| A | DE-A-3 906 891 (P.UEBERALL) <br><br> ----- | | RECHERCHIERTE SACHGEBIETE (Int. Cl.5 ) <br><br> A61L <br> C08J <br> A01N |

Der vorliegende Recherchenbericht wurde für alle Patentansprüche erstellt

| Recherchenort | Abschlußdatum der Recherche | Prüfer |
|---|---|---|
| DEN HAAG | 26 MAERZ 1992 | G COUSINS-VAN STEEN |